# EUROPEAN PATENT APPLICATION

(11) **EP 4 768 008 A1**
(43) Date of publication of application: **01.07.2026**
(21) Application number: 24884653.7
(22) Date of filing: 28.10.2024
(51) Int. Cl.: A61K 6/78, A61C 13/083, A61K 6/60, A61K 6/818

(54) **ZIRCONIA MODIFICATION SOLUTION AND USE THEREOF**

(30) Priority: 30.10.2023 CN 202311420248
(71) Applicant: Aidite (Qinhuangdao) Technology Co., Ltd., Qinhuangdao, Hebei 066004 (CN)
(72) Inventor: WU, Haiyan, Qinhuangdao Hebei 066004 (CN); AHOU, Shenggang, Qinhuangdao Hebei 066004 (CN); LI, Xiaoyu, Qinhuangdao Hebei 066004 (CN); GAO, Yang, Qinhuangdao Hebei 066004 (CN)
(74) Representative: Vogelbruch, Keang
(86) International application number: PCT/CN2024/127719
(87) International publication number: WO 2025/092650

(57) **Abstract**

Disclosed are a zirconia modification solution and use thereof. Based on a total mass percentage content of 100 wt%, the zirconia modification solution comprises: 30-80 wt% of a color-masking component, and a balance of a colored component. The zirconia modification solution exhibits a penetration rate of ≤ 20% into dental restorations, and a reduction in light transmittance of dental restorations by ≥ 20%. The zirconia modification solution achieves a color-masking effect without affecting the aesthetics of dental restorations. By controlling the contents of the color-masking component and the colored component, the color-masking layer can mask dark-colored abutment teeth, discolored teeth, or metal abutments, and is prevented from being visible through the surface of the zirconia dental restorations, thereby reducing the restoration effect. This achieves a color-masking effect without affecting the aesthetics of dental restorations.

## Description

### CROSS REFERENCE TO RELATED APPLICATION

The present application claims priority to Chinese Patent Application No. CN202311420248.9, filed with the China National Intellectual Property Administration on October 30, 2023, and entitled "ZIRCONIA MODIFICATION SOLUTION AND USE THEREOF", the disclosure of which is incorporated herein in its entirety by reference.

### TECHNICAL FIELD

The present application relates to the technical field of dental materials, particularly to a zirconia modification solution and use thereof.

### BACKGROUND ART

With improvements of living standards, people's demands for dental restorations not only focuses on restoring the chewing function of dentures, but also pays more attention to the aesthetics after denture restoration. The developments of zirconia ceramic fabrication technology have led to increasing use of aesthetically pleasing ceramics in the denture restorations. The aesthetics of dentures require that the color and transparency of a restoration material should be closely resemble and harmonize with natural teeth. However, aesthetic ceramics, due to their excellent transparency, have a disadvantage of insufficient color-masking ability in scenarios such as small thicknesses, darker color of abutment teeth, and implant-supported restorations, thereby leading to the colors of abutment teeth or metal abutments leaking from the inside to outside, thus affecting the overall aesthetics of the restorations. Currently, commercially available zirconia modification solutions are generally divided into a coating type and a penetrating type, wherein the coating type modification solution product improves the color-masking performance by coating the inner surface of a crown after a final sintering; however, the process of this coating type is complex, the operation is cumbersome, and the cost is high. A penetrating type modification solution product is used by brushing before the final sintering of the restoration, but the penetrating modification solution product in the prior art has a drawback of high penetration depth, leading to an unnatural chalky white color after the final sintering, which is visible through the crown, severely affecting the overall restoration effect, especially in use of ultra-thin restorations such as clinical veneers, failing to achieve an aesthetic effect.

CN 108703890A discloses a color-masking zirconia restoration, formed by adding one or more layers of a stained color-masking layer, a coated color-masking layer, and a color-bonding layer to the fitting surface of the zirconia restoration. However, the color-masking zirconia restoration requires the use of a multi-layered composite color-masking method to achieve a color-masking effect, which is cumbersome and highly dependent on a skill level of an operator. At the same time, too many color-masking layers may lead to excessive tooth preparation in clinical practice, which is not conducive to clinical application and promotion.

CN 110314001A discloses an opacity imparting solution, which is a liquid material that can only adjust transparency by applying on a part of a zirconia crown having high transparency, without coloring. The opacity imparting solution of the application is used for a prosthesis device cut and machined from a dental zirconia for cutting and machining, wherein, the opacity imparting solution includes: (a) 10 wt% to 39 wt% of a water-soluble aluminum compound and/or water-soluble lanthanum compound; (b) 60 wt% to 89 wt% of water; and (c) 1 wt% to 20 wt% of an organic solvent. However, highly transparent zirconia dental crowns have relatively high overall and lateral transparency. An opaque layer formed on the inner side of the crown after a treatment can easily penetrate the outer side of the crown, thus affecting overall permeability and color harmony of the entire restoration, reducing the inherent quality of the restorative material and the clinical restoration effect.

CN 109608233A discloses a technique for improving the permeability of a dental zirconia ceramic. Specifically, the technique includes a color-masking solution which has a formula comprising potassium nitrate, yttrium chloride, praseodymium nitrate, an alcohol, citric acid, gluconic acid, and water. Before the final sintering of zirconia ceramics, the color-masking solution is brushed onto the zirconia surface, followed by the final sintering. However, the color-masking solution has translucency-enhancing effect and cannot achieve an optimal color-masking effect on darker colored abutment teeth, discolored teeth, or metal abutments, causing the base color to be shown through the crown and severely affecting the aesthetics of the restorations.

Therefore, to address shortcomings of the prior art, there is an urgent need to provide a zirconia modification solution that can achieve a color-masking effect without affecting the overall aesthetics of dental restorations.

### SUMMARY

A purpose of the present application is to provide a zirconia modification solution and use thereof. By controlling the contents of a color-masking component and a colored component, the color-masking layer can mask dark-colored abutment teeth, discolored teeth, or metal abutments, thereby achieving a color-masking effect without affecting the aesthetics of dental restorations.

To achieve the purpose of the present application, the technical solutions used in the present application are:
In the first aspect, the present application provides a zirconia modification solution, and based on a total mass percentage content of 100 wt%, the zirconia modification solution includes 30-80 wt% of a color-masking component, and a balance of a colored component; and the zirconia modification solution exhibits a penetration rate of ≤ 20% into dental restorations, and a reduction light transmittance of dental restorations by ≥ 20%. The zirconia modification solution achieves a color-masking effect without affecting the aesthetics of dental restorations. In the present application, the reduction in light transmittance of dental restorations refers to the light transmittance of the dental zirconia restoration obtained after color-masking with the zirconia modification solution of the present application, relative to the light transmittance of the dental restoration before color-masking. The method of color-masking is shown below regarding the use of the zirconia modification solution.

The zirconia modification solution provided in the present application, by controlling the contents of the color-masking component and the colored component, can mask dark-colored abutment teeth, discolored teeth, or metal abutments with the color-masking layer, and prevent it from being visible through the surface of the zirconia dental restorations, thereby reducing the restoration effect. By using the zirconia modification solution, a color-masking effect is achieved without affecting the aesthetics of dental restorations. Meanwhile, by adjusting the color of the color-masking layer, it is avoided that the excessive transparency of zirconia causes the color of the color-masking layer to penetrate and affect the aesthetic effect.

The zirconia modification solution includes the color-masking component with a mass percentage content of 30wt% to 80 wt%, such as, 30 wt%, 40 wt%, 50 wt%, 60 wt%, 70 wt%, or 80 wt%, but is not limited to the listed values; other unlisted values within the range are also applicable.

If the content of the color-masking component in the zirconia modification solution is too low, dark-colored abutment teeth, discolored teeth, or metal abutments can be easily shown through the surface of the zirconia dental restorations, thereby reducing restoration effect. If the content is too high, the color of the color-masking layer can be shown, thereby affecting the aesthetics of the dental restorations.

The zirconia modification solution exhibits a penetration rate of ≤ 20% into dental restorations, for example, the penetration rate can be 20%, 18%, 15%, 12%, or 10%, but is not limited to the listed values; and other unlisted values within the range are also applicable.

A reduction in light transmittance of dental restorations is ≥ 20%, for example, the reduction can be 20%, 22%, 25%, 28%, or 30%, but is not limited to the listed values; and other unlisted values within the range are also applicable.

Preferably, the color-masking component includes an amphiphilic compound containing silicon element.

Preferably, the color-masking component includes any one or a combination of at least two of KH550, KH560, KH570, KH792, and DL602. Typical but non-limiting combinations include a combination of KH550 and KH560, a combination of KH570, KH792, and DL602, or a combination of KH550, KH560, KH570, KH792, and DL602.

The color-masking component in the present application works synergistically with the color component, forming a multiphase layer after high-temperature calcination. This layer of composite material has extremely low transparency, achieving a color-masking effect, ensuring that the use of the dental zirconia restorations meet aesthetic requirements; meanwhile, the color-masking layer has a low penetration depth, and will not penetrate to the outer surface of the zirconia restorations, thereby affecting the aesthetic effect thereof. The color-masking layer further has a adjustable color, thereby simulating the color of normal dentin and achieving a more biomimetic aesthetic effect.

Preferably, the colored component includes a soluble metal salt, a stabilizer, and a solvent.

Preferably, based on a mass percentage content of 100 wt% of the colored component, in the color component, the mass percentage content of the soluble metal salt is 0.5wt% to 58.18 wt%, the mass percentage content of the stabilizer is 0.13wt% to 16 wt%, and the balance is a solvent. The mass percentage content of the soluble metal salt is 0.5wt% to 58.18wt%, for example, it can be 0.5wt%, 1wt%, 7wt%, 14wt%, 20wt%, 28wt%, 50wt%, or 58.18wt%, but it is not limited to the listed values, and other unlisted values within the numerical range are also applicable.

The mass percentage content of the stabilizer is 0.13wt% to 16wt%, for example, the mass percentage content of the stabilizer can be 0.13wt%, 0.5wt%, 1.6wt%, 3.2wt%, 8.18wt%, 12wt%, or 16wt%, but it is not limited to the listed values, and other unlisted values within the numerical range are also applicable.

Preferably, the cation in the soluble metal salt includes any one or a combination of at least two of Fe³⁺, Ce³⁺, Er³⁺, Nd³⁺, Cr³⁺, Mn²⁺, Co²⁺, and Gd³⁺. Typical but non-limiting combinations include a combination of Fe³⁺ and Ce³⁺, a combination of Er³⁺, Nd³⁺, and Cr³⁺, a combination of Nd³⁺, Cr³⁺, Mn²⁺, Co²⁺, and Gd³⁺, or a combination of Fe³⁺, Ce³⁺, Er³⁺, Nd³⁺, Cr³⁺, Mn²⁺, Co²⁺, and Gd³⁺.

Preferably, the anion in the soluble metal salt includes any one or a combination of at least two of NO₃⁻, CH₃COO⁻, Cl⁻, C₅H₇O₅COO⁻, and SO₄²⁻. Typical but non-limiting combinations include a combination of NO₃⁻ and CH₃COO⁻, a combination of Cl⁻, C₅H₇O₅COO⁻, and SO₄²⁻, or a combination of NO₃⁻, CH₃COO⁻, Cl⁻, C₅H₇O₅COO⁻, and SO₄²⁻.

Preferably, the stabilizer includes any one or a combination of at least two of citric acid, tartaric acid, lactic acid, oxalic acid, malic acid, ascorbic acid, and polydextrose. Typical but non-limiting combinations include a combination of citric acid and tartaric acid, a combination of lactic acid, oxalic acid, malic acid, and ascorbic acid, or a combination of citric acid, tartaric acid, lactic acid, oxalic acid, malic acid, ascorbic acid, and polydextrose.

Preferably, the solvent includes any one or a combination of at least two of deionized water, pentaerythritol, ethylene glycol, 1,2-propanediol, 1,3-butanediol, 1,4-butanediol, polyethylene glycol, 1,6-hexanediol, neopentyl glycol, diethylene glycol, dipropylene glycol (I), trimethylolpropane, and glycerol. Typical but non-limiting combinations include a combination of deionized water and pentaerythritol, a combination of ethylene glycol, 1,2-propanediol, 1,3-butanediol, 1,4-butanediol, polyethylene glycol and 1,6-hexanediol, a combination of 1,6-hexanediol, neopentyl glycol, diethylene glycol, dipropylene glycol, trimethylolpropane and glycerol, or a combination of deionized water, pentaerythritol, ethylene glycol, 1,2-propanediol, 1,3-butanediol, 1,4-butanediol, polyethylene glycol, 1,6-hexanediol, neopentyl glycol, diethylene glycol, dipropylene glycol (I), trimethylolpropane and glycerol.

In the second aspect, the present application provides use of the zirconia modification solution according to the first aspect, wherein the zirconia modification solution is used for color-masking of dental restorations; and a method for color-masking of dental restorations using the zirconia modification solution includes following steps:
brushing and/or infiltrating the zirconia modification solution onto an inner surface of the dental restorations, followed by a sintering treatment.

By applying the zirconia modification solution provided in the present application to dental restorations, the transparency inside the dental restorations can be effectively reduced, the dark-colored abutment teeth, discolored teeth, and metal abutments can be masked. Due to a base color thereof that harmonizes with the color of the restorations, the problem of poor restorative results caused by being visible through the surface of the dental restorations can be avoided. Moreover, by adjusting the color of the color-masking layer formed by the zirconia modification solution, the color of the color-masking layer is prevented from being visible due to excessively high transparency of zirconia when the modification solution is used in highly transparent zirconia, thereby affecting the aesthetic effect.

Preferably, the temperature of the sintering treatment is 1,450 °C to 1,570 °C, for example, it can be 1,450 °C, 1,480 °C, 1,500 °C, 1,530 °C, or 1,570 °C, but is not limited to the listed values; and other unlisted values within the range are also applicable.

Preferably, the duration of the sintering treatment is 10 min to 150 min, for example, it can be 10 min, 50 min, 100 min, 120 min, or 150 min, but is not limited to the listed values; other unlisted values within the range are also applicable.

Compared with the prior art, the present application has following beneficial effects:
The zirconia modification solution provided in the present application, by controlling the contents of the color-masking component and the colored component, can mask dark-colored abutment teeth, discolored teeth, or metal abutments with the color-masking layer, and prevent it from being visible through the surface of the zirconia dental restorations, thereby reducing the restoration effect. By using the zirconia modification solution, a color-masking effect is achieved without affecting the aesthetics of dental restorations; meanwhile, by adjusting the color of the color-masking layer, it is avoided that the excessive transparency of zirconia causes the color of the color-masking layer to penetrate and affect the aesthetic effect.

### BRIEF DESCRIPTION OF DRAWINGS

FIG. 1 is an optical image of the dental zirconia restoration provided in Test Example 3 of the present application;
FIG. 2 is an optical image of the dental restoration provided in Test Example 3 of the present application;
FIG. 3 is an optical image of the dental zirconia restoration with internal filling of dark gray modeling clay provided in Test Example 3 of the present application; and
FIG. 4 is an optical image of the dental restoration with internal filling of dark gray modeling clay provided in Test Example 3 of the present application.

### DETAILED DESCRIPTION OF EMBODIMENTS

The technical solution of the present application will be further described below through specific embodiments. Those skilled in the art should understand that these embodiments are merely intended to assist in understanding the present application and should not be construed as specific limitations on the present application.

### Example 1

The present example provided a zirconia modification solution, wherein, based on a total mass percentage content of 100 wt%, the zirconia modification solution included: 50 wt% of a color-masking component, and a balance of a colored component; the color-masking component was KH550; based on a mass percentage content of 100 wt%, the colored component included 14 wt% of Fe(NO₃)₃, 3.2 wt% of citric acid, and a balance of deionized water.

### Example 2

The present example provided a zirconia modification solution, wherein, based on a total mass percentage content of 100 wt%, the zirconia modification solution included: 60 wt% of a color-masking component, and a balance of a colored component; the color-masking component was KH560; based on a mass percentage content of 100 wt%, the colored component included 0.5 wt% of Fe(NO₃)₃, 0.13 wt% of citric acid, and a balance of ethylene glycol.

### Example 3

The present example provided a zirconia modification solution, wherein, based on a total mass percentage content of 100 wt%, the zirconia modification solution included: 45 wt% of a color-masking component, and a balance of a colored component; the color-masking component was KH570; based on a mass percentage content of 100 wt%, the colored component included 58.18 wt% of FeCl₃, 8.18 wt% of lactic acid, and a balance of glycerol.

### Example 4

The present example provided a zirconia modification solution. The difference from Example 1 was that the mass percentage content of the color-masking component was adjusted to 30 wt%, with the balance of the colored component, and the rest were the same as in Example 1.

### Example 5

The present example provided a zirconia modification solution. The difference from Example 1 was that the mass percentage content of the color-masking component was adjusted to 80 wt%, with the balance of the colored component, and the rest were the same as in Example 1.

### Example 6

The present example provided a zirconia modification solution. The difference from Example 1 was that the mass percentage content of Fe(NO₃)₃ in the colored component was adjusted to 28wt%, the mass percentage content of citric acid was adjusted to 0.08wt%, with the balance of deionized water, and the rest were the same as in Example 1.

### Example 7

The present example provided a zirconia modification solution. The difference from Example 1 was that the mass percentage content of Fe(NO₃)₃ in the colored component was adjusted to 70wt%, the mass percentage content of citric acid was adjusted to 20wt%, with the balance of deionized water, and the rest were the same as in Example 1.

### Example 8

The present example provided a zirconia modification solution. The difference from Example 1 was that no citric acid was added to the colored component, and the mass percentage content of Fe(NO₃)₃ was adaptively adjusted to 50wt%, and the rest were the same as in Example 1.

### Example 9

The present example provided a zirconia modification solution, wherein, based on a total mass percentage content of 100 wt%, the zirconia modification solution included: 40 wt% of a color-masking component, and a balance of a colored component; the color-masking component was KH570; based on a mass percentage content of 100 wt%, the colored component included: 16 wt% of FeCl₃, 4.5 wt% of lactic acid, 67 wt% of ethylene glycol, 6 wt% of 1,2-propanediol, 3 wt% of 1,3-butanediol, 3 wt% of 1,4-butanediol, 0.3 wt% of polyethylene glycol, and 0.2 wt% of 1,6-hexanediol.

### Example 10

The present example provided a zirconia modification solution, wherein, based on a total mass percentage content of 100 wt%, the zirconia modification solution included: 30 wt% of a color-masking component, and a balance of a colored component; the color-masking component included: 50 wt% of KH550 and 50 wt% of KH560; based on a mass percentage content of 100 wt%, the colored component included: 14 wt% of Fe(NO₃)₃, 2 wt% of Ce(CH₃COO)₃, 2.2 wt% of citric acid, 1 wt% of tartaric acid, 40.8 wt% of deionized water, and 40 wt% of pentaerythritol.

### Example 11

The present example provided a zirconia modification solution, wherein, based on a total mass percentage content of 100 wt%, the zirconia modification solution included: 80 wt% of a color-masking component and a balance of a colored component; based on a mass percentage content of 100 wt%, the color-masking component included: 40wt% of DL602, 30 wt% of KH570, and 30 wt% of KH792; based on a mass percentage content of 100 wt%, the colored component included: 5 wt% of Cr₂(SO₄)₃, 8 wt% of ErCl₃, 1 wt% of Nd(C₅H₇O₅COO)₃, 2.2 wt% of lactic acid, 0.5 wt% of oxalic acid, 0.5 wt% of malic acid, 40 wt% of 1,2-propanediol, 22.8 wt% of 1,3-butanediol, and 20 wt% of 1,4-butanediol.

### Example 12

The present example provided a zirconia modification solution, wherein, based on a total mass percentage content of 100 wt%, the zirconia modification solution included: 50 wt% of a color-masking component and a balance of a colored component; based on a mass percentage content of 100 wt%, the color-masking component included: 50 wt% of KH550, 25 wt% of KH570, 15 wt% of KH792, and 10 wt% of DL602; based on a mass percentage content of 100 wt%, the colored component included: 5 wt% of Cr₂(SO₄)₃, 5 wt% of Mn(CH₃COO)₂, 8wt% of Co(NO₃)₃, 8 wt% of NdCl₃, and 2 wt% of Gd(C₅H₇O₅COO)₃, 0.04 wt% of malic acid , 0.04 wt% of ascorbic acid, 30 wt% of 1,6-hexanediol, 20 wt% of neopentyl glycol, 10 wt% of diethylene glycol, 10 wt% of dipropylene glycol, 1 wt% of trimethylolpropane, and 0.92 wt% of glycerol.

### Example 13

The present example provided a zirconia modification solution, wherein, based on a total mass percentage content of 100 wt%, the zirconia modification solution included: 50 wt% of a color-masking component and a balance of a colored component; based on a mass percentage content of 100 wt%, the color-masking component included: 70wt% of KH550, 10wt% of KH560, 10wt% of KH570, 5wt% of KH792, and 5wt% of DL602; based on a mass percentage content of 100 wt%, the colored component included 35wt% of Fe(NO₃)₃, 5wt% of Cr(NO₃)₃, 5wt% of Mn(NO₃)₂, 8wt% of Er(NO₃)₃, 8wt% of Co(NO₃)₃, 6wt% of Nd(NO₃)₃, 1wt% of Ce(NO₃)₃, 2wt% of Gd(NO₃)₃, 10wt% of lactic acid, 5wt% of malic acid, 3wt% of oxalic acid, 2wt% of ascorbic acid, 8wt% of deionized water, 0.5wt% of pentaerythritol, 0.5wt% of ethylene glycol, 0.1wt% of 1,2-propanediol, 0.1wt% of 1,3-butanediol, 0.1wt% of 1,4-butanediol, 0.1wt% of polyethylene glycol, 0.1wt% of 1,6-hexanediol, 0.1wt% of neopentyl glycol, 0.1 wt% of diethylene glycol, 0.1 wt% of dipropylene glycol, 0.1 wt% of trimethylolpropane, and 0.1 wt% of glycerol.

### Comparative Example 1

The present comparative example provided a zirconia modification solution. The difference from Example 1 was that the mass percentage content of the color-masking component was adjusted to 20 wt%, with the balance of the colored component, and the rest were the same as in Example 1.

### Comparative Example 2

The present comparative example provided a zirconia modification solution. The difference from Example 1 was that the mass percentage content of the color-masking component was adjusted to 90 wt%, with the balance of the colored component, and the rest were the same as in Example 1.

### Comparative Example 3

The present comparative example provided a dental restoration, wherein the dental restoration was a dental restoration obtained by only subjecting a sintering treatment at 1,480 °C for 100 min.

Test Example 1
penetration rate test: 15 mm × 10 mm × 8 mm dental restorations were immersed in the zirconia modification solutions provided in Examples 1 to 13 and Comparative Examples 1 and 2. After 1 s, the dental restorations were taken out, and the excess liquid on surface was wiped off, and the dental restorations were subjected to air drying at room temperature for 10 min, followed by drying at 120 °C for 30 min before being taken out. The dental restorations were then cut in half along the long sides using a polishing bur, and the cut surfaces were finished and polished to a smooth finish by sequentially using 800-mesh and 2000-mesh sandpapers. The polished dental restorations were then subjected to sintering at 1480 °C for 100 min with the cut surfaces facing down to obtain dental zirconia restorations. Penetrations were observed using a high-resolution microscope, and the penetration rates were calculated. Results are shown in Table 1.

### Test Example 2

Light transmittance test: One side of 15mm × 10mm × 8mm dental restorations were brushed with the zirconia modification solutions provided in Examples 1 to 13 and Comparative Examples 1 and 2. The dental restorations were subjected to air-drying at room temperature for 10 min, followed by drying in an oven at 120 °C for 5 min before turning over, then drying for another 5 min before turning over again, followed by drying at 180 °C for 10 min before being taken out. The dental restorations were then subjected to a sintering treatment at 1480 °C for 100 min to obtain dental zirconia restorations. The light transmittance was measured using a haze meter. Comparative Example 3 served as a control and was not brushed with the zirconia modification solution. The light transmittance was measured using a haze meter. The reduction in light transmittance of dental zirconia restorations obtained from Examples 1 to 12 and Comparative Examples 1 and 2 relative to the dental restoration in Comparative Example 3 was calculated. Results are shown in Table 1.

### Test Example 3

Color-masking effect, aesthetics of restoration, and solution stability tests: The zirconia modification solutions provided in Examples 1 to 13 and Comparative Examples 1 and 2 were uniformly brushed onto the inner surfaces of 1.2 mm thick anterior tooth restorations. The anterior tooth restorations were subjected to air drying at room temperature for 10 min, followed by drying in an oven at 120 °C for 10 min. The anterior teeth restorations were then subjected to a sintering treatment at 1480 °C for 100 min to obtain dental zirconia restorations. Compared to the dental restoration in Comparative Example 3, when the interiors of the restorations were filled with dark gray modeling clay (simulating a dark-colored abutment teeth or metal abutment), the color of the dark-colored abutment teeth inside the restoration will not significantly penetrate the outer surface of the restoration, indicating good color-masking effect; otherwise, the color-masking effect is poor. Compared to the dental restoration in Comparative Example 3, when the color of the color-masking layer on the inner surface of the restoration will not significantly penetrate the outer surface of the restoration, indicating good aesthetics of restoration; otherwise, the aesthetics of restoration was poor. If the zirconia modification solution produced flocculation or precipitation after being left in a sealed state for more than 10 min, the solution was considered unstable; otherwise, it was stable. Results are shown in Table 1.

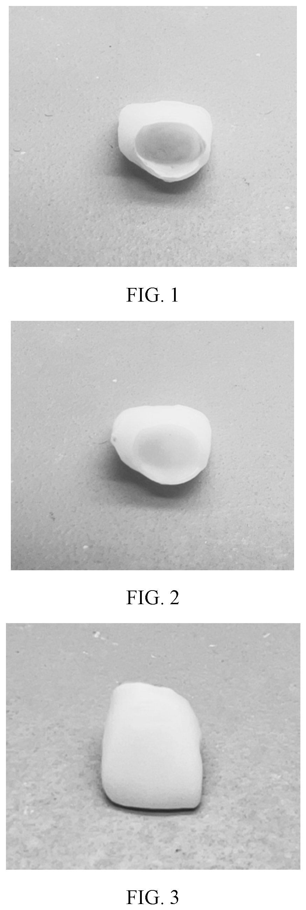

The optical image of the dental zirconia restoration obtained in Example 1 is shown in FIG. 1, and the optical image of the dental restoration obtained in Comparative Example 3 is shown in FIG. 2. The inner surface of the dental zirconia restoration brushed with zirconia modification solution can form a color-masking layer with a certain color effect. The color of the color-masking layer does not significantly penetrate the outer surface of the restoration, and the color of the outer surface of the restoration is basically unaffected, resulting in good aesthetic appearance of the restoration. In contrast, there is no significant difference in the optical properties between the inner surface and the outer surface of the dental restoration that have not been brushed with zirconia modification solution. The dental restoration has good aesthetic appearance but cannot achieve a color-masking effect.

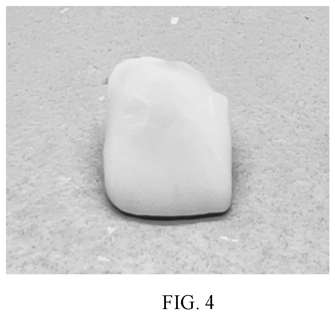

The optical image of the dental zirconia restoration obtained in Example 1, with interior filled with dark gray modeling clay, is shown in FIG. 3. The color of the dark-colored abutment tooth inside the restoration does not significantly penetrate the outer surface of the restoration, indicating a good color-masking effect. The optical image of the dental restoration obtained in Comparative Example 3, with interior filled with dark gray modeling clay, is shown in FIG. 4. The color of the dark-colored abutment tooth inside the restoration significantly penetrates the outer surface of the restoration, indicating a poor color-masking effect.

**Table 1**

| Name | Penetration Rate (%) | Reduction in Light Transmittance (%) | Color-masking Effect | Aesthetics of Restoration | Solution Stability |
|---|---|---|---|---|---|
| Example 1 | 11.00 | 28.53 | Good | Good | Stable |
| Example 2 | 7.17 | 22.77 | Good | Good | Stable |
| Example 3 | 18.33 | 67.20 | Good | Good | Stable |
| Example 4 | 19.24 | 26.77 | Good | Good | Stable |
| Example 5 | 19.95 | 32.74 | Good | Good | Stable |
| Example 6 | 16.84 | 30.04 | Good | Poor | Unstable |
| Example 7 | 20.00 | 71.41 | Good | Poor | Unstable |
| Example 8 | 18.21 | 59.87 | Good | Good | Unstable |
| Example 9 | 15.63 | 27.65 | Good | Good | Stable |
| Example 10 | 19.05 | 25.61 | Good | Good | Stable |
| Example 11 | 19.78 | 31.23 | Good | Good | Stable |
| Example 12 | 15.33 | 32.35 | Good | Poor | Unstable |
| Example 13 | 19.96 | 69.87 | Good | Poor | Unstable |
| Comperative Example 1 | 22.00 | 18.62 | Good | Poor | Stable |
| Comperative Example 2 | 25.00 | 34.59 | Good | Poor | Stable |
| Comperative Example 3 | / | / | Poor | Poor | / |

It can be seen from Table 1 that using the zirconia modification solution provided in the present application for color-masking of dental restorations avoids the problem of poor restoration effects caused by the color-masking layer being visible through the surface of the dental zirconia restoration, while not affecting the aesthetic effect.

As can be seen from the comparison of Examples 1-5, 10-11 and Comparative Examples 1 and 2, the contents of the color-masking component and the colored component in the zirconia modification solution should be limited to reasonable ranges. Too low or too high content of the color-masking component will lead to poor color-masking effect or the color of the color-masking layer to penetrate (an penetration rate exceeding 20% will lead to the color of the color-masking layer to penetrate), thus affecting the aesthetic effect. As can be seen from the comparison of Example 1 and Examples 6, 7, 12, and 13, the contents of the soluble metal salt and the stabilizer in the colored components also needs to be controlled within reasonable ranges. Otherwise, it will cause the color of the color-masking layer to be too white and penetrate the restoration, resulting in an increase in the brightness of the restoration, or the color of the color-masking layer penetrates the restoration, resulting in an unnatural color, thereby affecting the aesthetics of the dental zirconia restoration. The comparison between Example 1 and Example 8 shows that the absence of the stabilizer in the colored component causes hydrolysis reactions of cations, resulting in flocculent precipitates in the solution, affecting the color-masking effect and color uniformity.

The comparison between Example 1 and Comparative Example 3 shows that the inner surface of the dental restorations cannot achieve the color-masking effect without brushing zirconia modification solution.

In summary, the zirconia modification solution provided in the present application, by controlling the contents of the color-masking component and the colored component, can mask dark-colored abutment teeth, discolored teeth, or metal abutments with the color-masking layer, and prevent it from being visible through the surface of the zirconia dental restorations, thereby reducing the restoration effect. By using the zirconia modification solution, a color-masking effect is achieved without affecting the aesthetics of dental restorations; meanwhile, by adjusting the color of the color-masking layer, it is avoided that the excessive transparency of zirconia causes the color of the color-masking layer to penetrate and affect the aesthetic effect. The above descriptions are merely specific embodiments of the present application, but the scope of protection of the present application is not limited thereto. Those skilled in the art should understand that any variations or substitutions that can be easily conceived by those skilled in the art within the technical scope disclosed in the present application will fall within the scope of protection and application of the present application.

## Claims

1. A zirconia modification solution, **characterized in that**, based on a total mass percentage content of 100 wt%, the zirconia modification solution comprises 30-80 wt% of a color-masking component, and a balance of a colored component; and
the zirconia modification solution exhibits a penetration rate of ≤ 20% into dental restorations, and a reduction in light transmittance of dental restorations by ≥ 20%.

2. The zirconia modification solution according to claim 1, **characterized in that** the zirconia modification solution achieves a color-masking effect without affecting the aesthetics of dental restorations.

3. The zirconia modification solution according to claim 1 or 2, **characterized in that** the color-masking component comprises an amphiphilic compound containing silicon element.

4. The zirconia modification solution according to claim 1 or 2, **characterized in that** the color-masking component comprises any one or a combination of at least two of KH550, KH560, KH570, KH792, and DL602.

5. The zirconia modification solution according to claim or 4, **characterized in that** the color-masking component comprises a combination of KH550 and KH560, a combination of KH570, KH792, and DL602, or a combination of KH550, KH560, KH570, KH792, and DL602.

6. The zirconia modification solution according to claim 1 or 2, **characterized in that** the colored component comprises a soluble metal salt, a stabilizer, and a solvent.

7. The zirconia modification solution according to claim 6, **characterized in that**, based on a mass percentage content of 100 wt% of the colored component, in the color component, the mass percentage content of the soluble metal salt is 0.5wt% to 58.18 wt%, the mass percentage content of the stabilizer is 0.13wt% to 16 wt%, and the balance is solvent.

8. The zirconia modification solution according to claim 6, **characterized in that** cation in the soluble metal salt comprises any one or a combination of at least two of Fe³⁺, Ce³⁺, Er³⁺, Nd³⁺, Cr³⁺, Mn²⁺, Co²⁺, and Gd³⁺.

9. The zirconia modification solution according to claim 8, **characterized in that** the cation in the soluble metal salt comprises a combination of Fe³⁺ and Ce³⁺, a combination of Er³⁺, Nd³⁺, and Cr³⁺, a combination of Nd³⁺, Cr³⁺, Mn²⁺, Co²⁺, and Gd³⁺, or a combination of Fe³⁺, Ce³⁺, Er³⁺, Nd³⁺, Cr³⁺, Mn²⁺, Co²⁺, and Gd³⁺.

10. The zirconia modification solution according to claim 6, **characterized in that** anion in the soluble metal salt comprises any one or a combination of at least two of NO₃⁻, CH₃COO⁻, Cl⁻, C₅H₇O₅COO⁻, and SO₄²⁻.

11. The zirconia modification solution according to claim 10, **characterized in that** the anion in the soluble metal salt comprises a combination of NO₃⁻ and CH₃COO⁻, a combination of Cl⁻, C₅H₇O₅COO⁻, and SO₄²⁻, or a combination of NO₃⁻, CH₃COO⁻, Cl⁻, C₅H₇O₅COO⁻, and SO₄²⁻.

12. The zirconia modification solution according to claim 7, **characterized in that** cation in the soluble metal salt comprises any one or a combination of at least two of Fe³⁺, Ce³⁺, Er³⁺, Nd³⁺, Cr³⁺, Mn²⁺, Co²⁺, and Gd³⁺.

13. The zirconia modification solution according to claim 7, **characterized in that** anion in the soluble metal salt comprises any one or a combination of at least two of NO₃⁻, CH₃COO⁻, Cl⁻, C₅H₇O₅COO⁻, and SO₄²⁻.

14. The zirconia modification solution according to claim 6, **characterized in that** the stabilizer comprises any one or a combination of at least two of citric acid, tartaric acid, lactic acid, oxalic acid, malic acid, ascorbic acid, and polydextrose.

15. The zirconia modification solution according to claim or 14, **characterized in that** the stabilizer comprises a combination of citric acid and tartaric acid, a combination of lactic acid, oxalic acid, malic acid, and ascorbic acid, or a combination of citric acid, tartaric acid, lactic acid, oxalic acid, malic acid, ascorbic acid, and polydextrose.

16. The zirconia modification solution according to claim 7, **characterized in that** the stabilizer comprises any one or a combination of at least two of citric acid, tartaric acid, lactic acid, oxalic acid, malic acid, ascorbic acid, and polydextrose.

17. The zirconia modification solution according to claim 6, **characterized in that** the solvent comprises any one or a combination of at least two of deionized water, pentaerythritol, ethylene glycol, 1,2-propanediol, 1,3-butanediol, 1,4-butanediol, polyethylene glycol, 1,6-hexanediol, neopentyl glycol, diethylene glycol, dipropylene glycol, trimethylolpropane, and glycerol.

18. The zirconia modified solution according to claim 17, **characterized in that** the solvent comprises a combination of deionized water and pentaerythritol, a combination of ethylene glycol, 1,2-propanediol, 1,3-butanediol, 1,4-butanediol, polyethylene glycol and 1,6-hexanediol, a combination of 1,6-hexanediol, neopentyl glycol, diethylene glycol, dipropylene glycol, trimethylolpropane and glycerol, or a combination of deionized water, pentaerythritol, ethylene glycol, 1,2-propanediol, 1,3-butanediol, 1,4-butanediol, polyethylene glycol, 1,6-hexanediol, neopentyl glycol, diethylene glycol, dipropylene glycol, trimethylolpropane and glycerol.

19. The zirconia modified solution according to claim 7, **characterized in that** the solvent comprises any one or a combination of at least two of deionized water, pentaerythritol, ethylene glycol, 1,2-propanediol, 1,3-butanediol, 1,4-butanediol, polyethylene glycol, 1,6-hexanediol, neopentyl glycol, diethylene glycol, dipropylene glycol, trimethylolpropane, and glycerol.

20. Use of the zirconia modification solution according to any one of claims 1 to 19, **characterized in that** the zirconia modification solution is used for color-masking of dental restorations; and a method for color-masking of dental restorations using the zirconia modification solution comprises the following steps:
brushing and/or infiltrating the zirconia modification solution onto the inner surface of the dental restorations, and then performing a sintering treatment.

21. The use according to claim 20, **characterized in that** the temperature of the sintering treatment is 1,450 °C to1,570 °C.

22. The use according to claim 20 or 21, **characterized in that** the duration of the sintering treatment is 10 min to 150 min.

23. A dental zirconia restoration, **characterized in that**, the dental zirconia restoration comprises a dental restoration and a color-masking layer located on the inner surface of the dental restoration; and the color-masking layer is formed by sintering the zirconia modification solution according to any one of claims 1 to 19.
